# EUROPEAN PATENT SPECIFICATION

(11) **EP 4 380 642 B1**
(45) Date of publication and mention of the grant of the patent: **21.01.2026**
(21) Application number: 22758368.9
(22) Date of filing: 04.08.2022
(51) Int. Cl.: A61M 1/00, A61B 17/22, A61M 3/02

(54) **CONTINUOUS SUCTION AND REPERFUSION MECHANISM**
KONTINUIERLICHER SAUG- UND REPERFUSIONSMECHANISMUS
MÉCANISME CONTINU D'ASPIRATION ET DE REPERFUSION

(30) Priority: 06.08.2021 US 202163230268 P
(43) Date of publication of application: 12.06.2024
(73) Proprietor: Boston Scientific Medical Device Limited, Galway (IE)
(72) Inventor: SHARMA, Deepak Kumar, Muzaffarnagar (IN); SINGH, Rajivkumar, Thane (IN); SUKHATME, Mihir, Thane (IN)
(74) Representative: Vossius & Partner Patentanwälte Rechtsanwälte mbB
(86) International application number: PCT/US2022/039436
(87) International publication number: WO 2023/014891

(56) References cited:
- US-A1- 2003 069 549
- US-A1- 2005 084 395

## Description

### TECHNOLOGY FIELD

The present application relates generally to a thrombectomy system, and in particular, to a thrombectomy system with continuous suction and reperfusion.

### BACKGROUND

A wide variety of intracorporeal medical devices have been developed for medical use, for example, intravascular use. Some of these devices include guidewires, catheters, and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices.
US 2003/069549 A1 discloses a system for irrigation and aspiration within a localized region of the body comprising a fluid exchange apparatus having an irrigation reservoir and irrigation lumen and having means for controlled delivery of fluid from the irrigation reservoir through the irrigation lumen to a target site. A catheter element comprises the irrigation lumen, wherein the irrigation lumen terminates with at least one irrigation port at a distal end of the catheter element and the aspiration lumen terminates with at least one aspiration lumen such that fluid volume exchange occurs between the at least one irrigation port and the at least one aspiration port.
US 2005/084395 A1 discloses a vacuum driven pump comprising: a cylinder casing; a first chamber and a second chamber separated by a separating wall; a first piston and a second piston linked by a rod which goes through said separating wall; a first port associated with said first chamber and located between said separating wall and said first piston; a second port associated with said second chamber and located between said separating wall and said second piston; a first inlet valve to allow fluid to enter said first chamber and a second inlet valve to allow fluid to enter said second chamber wherein said first inlet valve and said second inlet valve are located at opposite ends of said cylinder casing; a first outlet valve to allow fluid to exit said first chamber and a second outlet valve to allow fluid to exit said second chamber wherein said first outlet valve is located on the same end of said cylinder as said first inlet valve and said second outlet valve is located on the same end of said cylinder as said second inlet valve; a vacuum source; a liquid source; and a vacuum controller.

### SUMMARY

This disclosure provides design, material, manufacturing methods, and use alternatives for medical devices. The invention relates to a suction device as defined in claim 1. Further embodiments of the invention are specified in the dependent claims.

A first example includes a suction device comprising a barrel defining a cavity and extending from a first end to a second end; a first fluid inlet adjacent the first end of the barrel and a second fluid inlet adjacent the second end of the barrel; a first fluid outlet adjacent the first end of the barrel and a second fluid outlet adjacent the second end of the barrel; a plunger slideably disposed within the cavity of the barrel; and a handle assembly operably coupled to the plunger. The actuation of the handle assembly is configured to move the plunger within the cavity between the first end and the second end of the barrel.

The handle assembly comprises a moveable first handle member pivotably coupled to a second handle member.

Further comprising a connecting member extending between the first handle member and the second handle member.

Additionally to the example above, wherein a portion of the first handle member is moveably disposed within a slot of the connecting member.

Wherein the second handle member is fixedly coupled to the connecting member.

Wherein the connecting member is fixedly secured to the barrel.

Alternatively or additionally to any of the examples above, in another example further comprising a biasing mechanism disposed within the connecting member.

Alternatively or additionally to any of the examples above, in another example wherein the biasing mechanism is configured to bias the first handle member towards the first end of the barrel.

Alternatively or additionally to any of the examples above, in another example wherein actuation of the first handle member is configured to move the plunger in a first direction and the biasing mechanism is configured to move the plunger in a second direction opposite the first direction.

Alternatively or additionally to any of the examples above, in another example wherein the plunger includes a magnetic material.

Alternatively or additionally to any of the examples above, in another example wherein the handle assembly is magnetically coupled to the plunger.

Alternatively or additionally to any of the examples above, in another example wherein the handle assembly comprises an annular portion disposed about an outer perimeter of the barrel.

Alternatively or additionally to any of the examples above, in another example wherein the annular portion includes a magnetic material.

Alternatively or additionally to any of the examples above, in another example wherein movement of the plunger within the cavity is configured to simultaneously draw fluid in through at least one of the first and second inlets and expel fluid through at least one of the first and second outlets.

Alternatively or additionally to any of the examples above, in another example wherein the plunger forms a fluid tight seal with an interior surface of the barrel.

Another example includes a suction device comprising: a barrel defining a cavity and extending from a first end to a second end; a first fluid inlet adjacent the first end of the barrel and a second fluid inlet adjacent the second end of the barrel;
a first fluid outlet adjacent the first end of the barrel and a second fluid outlet adjacent the second end of the barrel; a plunger slideably disposed within the cavity of the barrel; and a handle assembly operably coupled to the plunger. The actuation of the handle assembly is configured to move the plunger within the cavity between the first end and the second end of the barrel.

Alternatively or additionally to any of the examples above, in another example wherein the handle assembly comprises a moveable first handle member pivotably coupled to a second handle member.

Alternatively or additionally to any of the examples above, in another example further comprising a connecting member extending between the first handle member and the second handle member.

Alternatively or additionally to any of the examples above, in another example wherein a portion of the first handle member is moveably disposed within a slot of the connecting member.

Alternatively or additionally to any of the examples above, in another example further comprising a biasing mechanism disposed within the connecting member.

Alternatively or additionally to any of the examples above, in another example wherein the biasing mechanism is configured to bias the first handle member towards the first end of the barrel.

Alternatively or additionally to any of the examples above, in another example wherein actuation of the first handle member is configured to move the plunger in a first direction and the biasing mechanism is configured to move the plunger in a second direction opposite the first direction.

Alternatively or additionally to any of the examples above, in another example wherein the plunger includes a magnetic material.

Alternatively or additionally to any of the examples above, in another example wherein the handle assembly is magnetically coupled to the plunger.

Alternatively or additionally to any of the examples above, in another example wherein the handle assembly comprises an annular portion disposed about the outer perimeter of the barrel.

Alternatively or additionally to any of the examples above, in another example wherein the annular portion includes a magnetic material.

Alternatively or additionally to any of the examples above, in another example wherein movement of the plunger within the cavity is configured to simultaneously draw fluid in through at least one of the first and second inlets and expel fluid through at least one of the first and second outlets.

Another example incluses a suction device, comprising: a barrel defining a cavity and extending from a first end to a second end; a first fluid inlet adjacent the first end of the barrel and a second fluid inlet adjacent the second end of the barrel; a first fluid outlet adjacent the first end of the barrel and a second fluid outlet adjacent the second end of the barrel; a plunger slideably disposed within the cavity of the barrel, the plunger forming a fluid tight seal with an interior surface of the barrel; and a handle assembly operably coupled to the plunger. The handle assembly comprising: a first actuatable lever; a stationary member; and a connecting member extending between the first actuatable lever and the stationary member. Actuation of the first actuatable lever is configured to move the plunger within the cavity to simultaneously draw fluid in through at least one of the first and second inlets and expel fluid through at least one of the first and second outlets.

Alternatively or additionally to any of the examples above, in another example wherein the first actuatable lever comprises an annular first end, a gripping region, and an intermediate neck region disposed between the annular first end and the gripping region.

Alternatively or additionally to any of the examples above, in another example wherein the intermediate neck region is moveably disposed within a slot of the connecting member of the handle assembly.

Alternatively or additionally to any of the examples above, in another example wherein the annular first end is configured to extend about an outer perimeter of the barrel adjacent to the plunger.

Alternatively or additionally to any of the examples above, in another example, wherein the annular first end is magnetically coupled to the plunger.

Alternatively or additionally to any of the examples above, in another example further comprising a biasing mechanism disposed within the connecting member.

Alternatively or additionally to any of the examples above, in another example wherein the biasing mechanism is configured to bias the first actuatable lever towards the first end of the barrel.
Another example includes a suction device, comprising: a barrel defining a cavity and extending from a first end to a second end; a first fluid inlet adjacent the first end of the barrel and a second fluid inlet adjacent the second end of the barrel; a first fluid outlet adjacent the first end of the barrel and a second fluid outlet adjacent the second end of the barrel; a plunger slideably disposed within the cavity of the barrel, the plunger including a magnet and forming a fluid tight seal with an interior surface of the barrel; and a handle assembly operably coupled to the plunger. The handle assembly comprising: a first actuatable lever comprising a magnetic first end magnetically coupled to the plunger; a stationary member; a connecting member extending between the first actuatable lever and the stationary member; and a biasing mechanism disposed within the connecting member. Actuation of the first actuatable lever is configured to move the plunger in a first direction to simultaneously draw fluid in through at least one of the first and second inlets and expel fluid through at least one of the first and second outlets and the biasing mechanism is configured to move the first actuatable lever and the plunger in second direction opposite the first direction to simultaneously draw fluid in through at least one of the first and second inlets and expel fluid through at least one of the first and second outlets.

The above summary of some example embodiments is not intended to describe each disclosed embodiment or every implementation of the disclosure.

### BRIEF DESCRIPTION OF THE DRAWINGS

The invention may be more completely understood in consideration of the following detailed description of various embodiments in connection with the accompanying drawings, in which:
FIG. 1 is a side view of an illustrative suction device in a first configuration;
FIG. 2 is a cross-sectional view of the illustrative hand operated suction device of FIG. 1;
FIG. 3 is a cross-sectional view of the illustrative hand operated suction device of FIG. 1 in a second configuration;
FIG. 4 is a partial cross-sectional view of a portion of the illustrative hand operated suction device of FIG. 1; and
FIG. 5 is a schematic view of an illustrative thrombectomy system;
FIG. 6 is a cross-sectional view of an illustrative on/off valve
FIG. 7 is a perspective view of the actuation mechanism of the illustrative valve of FIG. 6; and
FIG. 8 is a cross-sectional view of another illustrative on/off valve.

While the invention is amenable to various modifications and alternative forms, specifics thereof have been shown by way of example in the drawings and will be described in detail. It should be understood, however, that the intention is not to limit aspects of the invention to the particular embodiments described. On the contrary, the intention is to cover all modifications, equivalents, and alternatives falling within the scope of the claims.

### DETAILED DESCRIPTION OF EXEMPLARY EMBODIMENTS

All numeric values are herein assumed to be modified by the term "about", whether or not explicitly indicated. The term "about" generally refers to a range of numbers that one of skill in the art would consider equivalent to the recited value (i.e., having the same function or result). In many instances, the term "about" may be indicative as including numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

Although some suitable dimensions, ranges, and/or values pertaining to various components, features and/or specifications are disclosed, one of skill in the art, incited by the present disclosure, would understand desired dimensions, ranges and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The detailed description and the drawings, which are not necessarily to scale, depict illustrative embodiments and are not intended to limit the scope of the invention. The illustrative embodiments depicted are intended only as exemplary. Selected features of any illustrative embodiment may be incorporated into an additional embodiment unless clearly stated to the contrary.

Thrombectomy catheters and systems may be used to remove thrombus, plaques, lesions, clots, etc. from veins or arteries. These devices may use devices to mechanically dislodge and remove the thrombus. Additionally, or alternatively, aspiration may be used to remove the thrombus. In some cases, a simple syringe may be used to aspirate thrombus, plaques, lesions, clots, etc. However, there may be several drawbacks to using syringes for aspiration. While syringes may have a familiar interface, they may not be intuitive to use for aspiration. Further, traditional syringes do not allow for continuous suction and provide limited blood suction capacity. A lack of continuous suction may require the clinician to empty the syringe multiple times during a procedure which may create a blockage within the catheter. Additionally, reperfusion of the aspirated blood may not be performed with a syringe which leads to blood loss. Further, a suction force of in the range of 18-20 pound-force (lbf) (80-89 Newtons) may be required which may be beyond ergonomic limits. In addition, the suction force may be limited to the capacity of the valve and may be in the range of 25 mm Hg. It is further contemplated that a simple syringe may not allow for control of the suction pressure. The use of a syringe for aspiration may require a dedicated person to operate the syringe as it requires two hands for operation. Additionally, the aspiration process may require multiple steps, including, but not limited to actuating the syringe, emptying the syringe, and reconnecting the syringe, which may increase the operation or procedure time. Alternative suction mechanisms may be desirable.

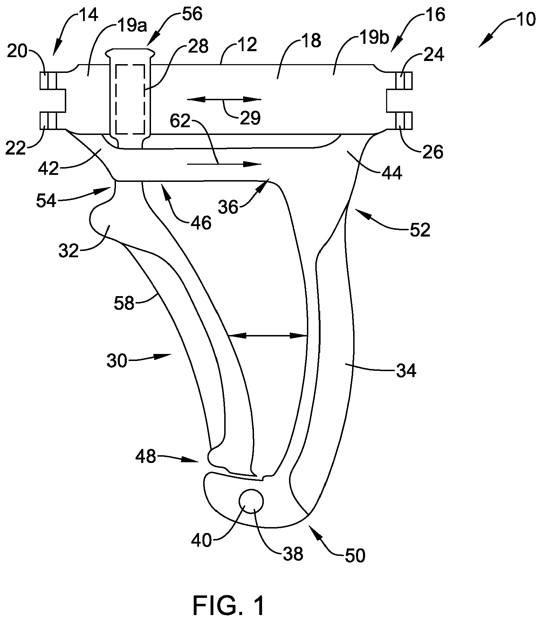

FIG. 1 is a side view of an illustrative suction device 10 that may be used with a thrombectomy catheter system or other medical device system. Generally, the suction device 10 may be hand operated or articulatable to simultaneously draw fluid into a cavity 18 and expel fluid from the cavity 18. The suction device 10 may be sized and shaped to be held within a user's hand, if so desired. The suction device 10 may include a barrel 12 extending from a first end 14 to a second end 16 and defining the cavity 18. The cavity 18 may define a volume in the range of about 60 cubic centimeters. However, the volume may be less than 60 cubic centimeters or greater than 60 cubic centimeters, as desired.

The barrel 12 may include a first fluid inlet 20 and a first fluid outlet 22 adjacent the first end 14 thereof. The barrel 12 may further include a second fluid inlet 24 and a second fluid outlet 26 adjacent the second end 16 thereof. The cavity 18 may be in fluid communication with each of the first and second inlets 20, 24 and the first and second outlets 22, 26. Further, the first and second inlets 20, 24 define openings which may be fluidly coupled to a fluid source while the first and second fluid outlets 22, 26 define openings which may be fluidly coupled to a fluid outlet. As will be described in more detail herein, as fluid is drawn in through one of the fluid inlets 20, 24 fluid is expelled through an opposing fluid outlet 22, 26

A plunger 28 may be slideably or movably disposed inside of the cavity 18. The plunger 28 may separate the cavity 18 into a first cavity portion 19a and a second cavity portion 19b. The plunger 28 may be configured to move towards the second end 16 of the barrel 12 and back towards the first end 14 of the barrel, as shown at arrow 29. The volumes of the first cavity portion 19a and the second cavity portion 19b may change as the plunger 28 is moved between the first and second ends 14, 16. For example, as the plunger 28 moves from the first end 14 towards the second end 16, volume of the first cavity portion 19a increases and the volume of the second cavity portion 19b decreases. When the plunger moves from the second end 16 towards the first end 14, the volume of the first cavity portion 19a decreases and the volume of the second cavity portion 19b increases.

The plunger 28 may be formed from rubber, silicone, or other material configured to provide a fluid tight-seal with an interior surface of the barrel 12. The plunger 28 may be operably coupled to a handle assembly 30 to move the plunger 28 within the cavity 18 of the barrel 12. The handle assembly 30 may include a first handle member 32, a second handle member 34, and a connecting member 36. The first handle member 32 may be a lever which is may be movably coupled to second handle member 34 and the connecting member 36. For example, a second end 50 of the second handle member 34 may be coupled to the first handle member 32 adjacent a second end thereof 48 via a hinge assembly 38. In some cases, the hinge assembly 38 may include a pin 40 configured to pivotably secure the first handle member 32 to the second handle member 34. However, other hinge mechanisms may be used, as desired. The first handle member 32 may be squeezed or otherwise moved towards to the second handle member 34 to move the plunger 28 within the cavity 18. Generally, the handle assembly 30 may be configured to allow a user to actuate the suction device 10 with a single hand. For example, the first handle member 32 may be configured to be held or gripped by the user's fingers while the second handle member 34 may be held in the user's palm which allows the handle assembly 30 and thus the suction device 10 to be controlled with a single hand. Other single hand gripping techniques may be used, as desired.

The connecting member 36 may extend between a first end 42 and a second end 44. The first end 42 of the connecting member 36 may be fixedly secured to the barrel 12 adjacent to the first end 14 thereof and a second end 44 of the connecting member 36 may be fixedly secured to the barrel 12 adjacent to the second end 16 thereof. A first end 52 of the second handle member 34 of the handle assembly 30 may be coupled to and extend from the second end 44 of the connecting member 36 at a non-parallel angle relative to a longitudinal axis of the connecting member 36. In some cases, the connecting member 36 and the second handle member 34 may be formed from a single monolithic structure, although this is not required.

A slot or channel 46 may extend through the connecting member 36 from a top surface to a bottom surface and between the first end 42 and the second end 44 of the connecting member 36. An intermediate neck region 54 of the first handle member 32 may be movably disposed within the channel 46. For example, as the first handle member 32 is moved towards the second handle member 34, the intermediate region 54 thereof is moved within the slot 46 towards the second end 16 of the barrel 12, as shown at arrow 62. The intermediate neck region 54 of the first handle member 32 may be positioned between an annular first end 56 and a gripping region 58. The annular first end 56 may be sized and shaped to extend about an outer perimeter of the barrel 12 adjacent to the plunger 28. The annular first end 56 may be formed from a magnetic material or have magnetic material embedded therein.

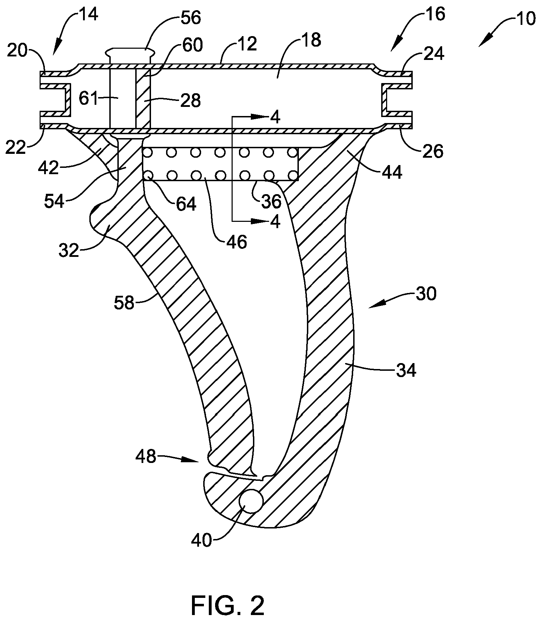

Referring additionally to FIG. 2, which illustrates a cross-sectional view of the illustrative suction device 10 of FIG. 1, the plunger 28 includes a magnet 60 disposed thereon. In some cases, the magnet 60 may form one end of the plunger 28 while a fluid-tight rubber 61 might form another end of the plunger 28. However, other configurations may be used, as desired. While the magnet 60 is illustrated as a single magnet, it is contemplated that the plunger 28 may include more than one magnet or a plurality of magnetic particles dispersed through the plunger 28. Further, the magnet 60 may be embedded in or extend about a perimeter (internal or external) of the plunger 28, as desired. The magnet 60 within the plunger 28 may be magnetically coupled with the magnetic annular first end 56 of the first handle member 32. For example, the magnet 60 and the first end 56 of the first handle member 32 may have opposing polarity. Thus, as the first handle member 32 is actuated and the first end 56 moved, the plunger 28 moves with first end 56 due to the magnetic coupling. The first end 56, and thus the plunger 28 may be configured to move towards the second end 16 of the barrel 12 as the user squeezes, or otherwise moves, the first handle member 32 towards the second handle member 34. It is further contemplated that the lever design of the first handle member 32 may provide a more ergonomic and intuitive design.

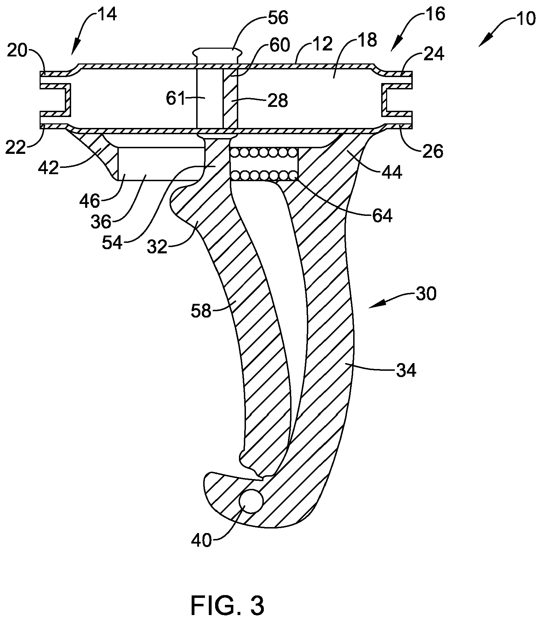

A biasing mechanism or spring 64 may be positioned within the connecting member 36. As the first handle member 32 is moved towards the second handle member 34, the intermediate neck region 54 pushes against the spring 64 and the spring 64 is biased into a compressed configuration, as shown in FIG. 3, which illustrates a cross-sectional view of the illustrative suction device 10 in a second configuration. When the user releases the biasing force on the first handle member 32, the spring 64 resumes its expanded configuration (e.g., FIG. 2) and moves the first handle member 32 (and thus its first end 56 and the plunger 28) back towards the first end 14 of the barrel 12 without user interaction. Said differently, the spring 64 is configure to bias the first handle member 32 towards the first end 14 of the barrel 12.

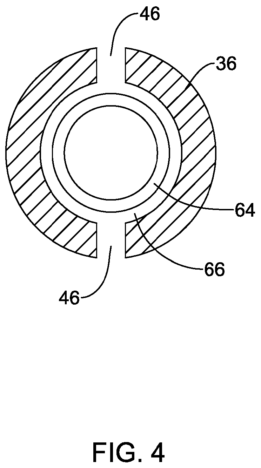

FIG. 4 is an illustrative cross-sectional view of the connecting member 36 with the spring 64 disposed therein, taken at line 4-4 of FIG. 2. The slot(s) 46 may extend through the top and bottom walls of the connecting member 36. The intermediate neck region 54 may be sized and shaped to be slidably disposed within the slots 46. The slots 46 may be in communication with a cavity 66 disposed within the connecting member 36. The cavity 66 may have a larger cross-sectional dimension than the slot(s) 46 such that the spring 64 is retained within the cavity 66 while allowing the intermediate neck region 54 of the first handle member 32 to slide within the slots 46.

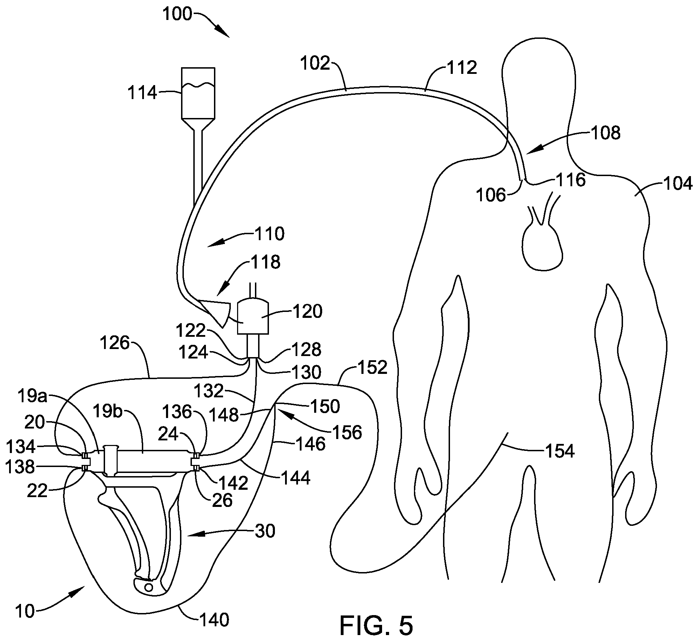

FIG. 5 is a schematic overview of an illustrative thrombectomy system 100 for removing thrombus, plaques, lesions, clots, etc. from veins or arteries including a suction device 10. The system 100 may include a thrombectomy catheter 102 which is configured to be advanced into a patient 104 to a target location. The thrombectomy catheter 102 may be a mechanical thrombectomy catheter which is configured to mechanically break up blockages. An illustrative thrombectomy catheter 102 is described in commonly assigned U.S. Patent Number 10,828,061, titled ACCESSORY DEVICES FOR USE WITH CATHETERS. However, the thrombectomy catheter 102 may take the form of other catheters, as desired. For example, the suction device 10 may be used with other catheters or devices where suction is desired. The thrombectomy catheter 102 may optionally be advanced through the vasculature over a guidewire 106.

A distal end region 108 of the catheter 102 may be configured to be advanced through the vasculature of the patient 104 to a target location while a proximal end region 110 may be configured to remain outside the body. While not explicitly shown, the proximal end region 110 may include a handle to facilitate actuation of the catheter 102, although this is not required. A lumen 112 may extend from the distal end region 108 to the proximal end region 110. In some cases, a fluid source 114, such as, but not limited to a saline bag, may be in fluid communication with the lumen 112. It is contemplated that the fluid source 114 may be used to prime the fluid circuit before use. The lumen 112 may be fluidly coupled to a distal opening 116 such that thrombus and/or blood may be aspirated from the target location. The proximal end region of the lumen 112 may be fluidly coupled to an inlet of an on/off valve 118. As will be described in more detail herein, the on/off valve 118 may be used to control a flow of blood from the patient 104 and a suction pressure.

The outlet of the valve 118 may be fluidly coupled to a filter 120. The filter 120 may be configured to remove debris, such as, but not limited to, thrombus, plaques, lesions, clots, etc. from the blood. The filter 120 may include a first outlet 122 fluidly coupled to a proximal end 124 of a first tube 126 and a second outlet 128 fluidly coupled to a proximal end 130 of a second tube 132. The distal ends 134, 136 of the first and second tubes 126, 132 may be fluidly coupled to the first inlet 20 and the second inlet 24, respectively, of the suction device 10. Each of the fluid outlets 22, 26 of the suction device 10 may be fluidly coupled to a proximal end 138 of a third tube 140 and a proximal end 142 of a fourth tube 144, respectively. The distal ends 146, 148 of the third and fourth tubes 140, 144 may be fluidly connected to a proximal end 150 of a reperfusion tube 152 via a Y-connector 156 or another device. The reperfusion tube 152 may include a distal end region 154 configured to be positioned within the body to reintroduce filtered blood back into the patient 104. As can be seen, each inlet 20, 24 of the suction device 10 is configured to receive blood or fluid coming from the patient 104 and each outlet 22, 26 of the suction device 10 is for blood or fluid to be perfused back to the patient 104. The thrombectomy system 100 creates a closed sterile environment which allows blood to be drawn from the patient, filtered, and safely perfused to the patient 104. This may reduce blood loss of the patient 104 during the procedure.

To aspirate or draw blood from the patient, the distal end region 108 of the catheter 102 may be positioned at the target location and the on/off valve 118 may be opened. The user may then grip the handle assembly 30 of the suction device 10 and move the first handle member 32 towards the second handle member 34. As the plunger 28 is moved towards the second end 16 of the barrel 12 (e.g., via the magnetic coupling between the first end 56 and the magnet 60), the fluid-tight seal between the plunger 28 and the interior surface of the barrel 12 creates a vacuum and fluid is drawn into the lumen of catheter 102 via the distal opening 116, through the filter 120, and into the cavity 18 of the barrel 12 through the first fluid inlet 20 (e.g., and into the first cavity portion 19a). As the plunger 28 moves towards the second end 16, any fluid that was drawn in through the second inlet 24 (e.g., fluid within the second cavity portion 19b) is pushed or expelled from the second fluid outlet 26. The user may then release the force on the first handle member 32 and the spring 64 pushes the first handle member 32 and thus the first end 56 and plunger 28 back towards the first end 14 of the barrel 12. As the plunger 28 moves towards the first end 14, any fluid that was drawn into the first cavity portion 19a through the first inlet 20 is pushed or expelled from the first fluid outlet 22. Further, as the plunger 28 moves towards the first end 14, a vacuum is created and fluid is drawn into the second cavity portion 19b of the barrel 12 through the second fluid inlet 24.

The suction device 10 may continue to provide suction as long as the user actuates the handle assembly 30 to continue to move the plunger 28 back and forth within the cavity 18. Continuous suction through repeated actuation of the first handle member 32 of the handle assembly 30 may provide a lower pressure ramp time (e.g., pressure may be quickly increased). Further, continuous suction may remove all debris stuck inside the catheter 102 and/or from the patient 104. It is contemplated that the suction force may be controlled by the stroke (or distance of travel) of the plunger 28. For example, the greater the distance the first handle member 32 is moved, the greater distance the plunger 28 will travel. Increased stroke or distance of travel may increase the suction force. Thus, the user may control the stroke volume through manipulation of the first handle member 32. It is contemplated that the suction force may be in the range of about 6 to 10 lbf. Further, as the plunger 28 draws in fluid as it moves in either direction, the suction device 10 may have increased suction capacity over a traditional syringe.

In some cases, the user may be required to actuate the handle assembly 30 more than one time before blood or fluid is drawn into the cavity 18 via either inlet 20, 24. However, the fluid circuit may be initially primed, for example, using saline, to prevent air from being introduced into the patient. As noted above, the blood or fluid passes through the filter 120 prior to entering the suction device 10. Thus, when the blood or fluid is reintroduced into the body via the reperfusion tube 152, the blood or fluid is substantially free from unwanted debris. Further, the debris may be removed without removal and/or reattachment of any components which may allow the procedure to be completed in less time than if a device needed to be removed and reattached throughout the procedure.

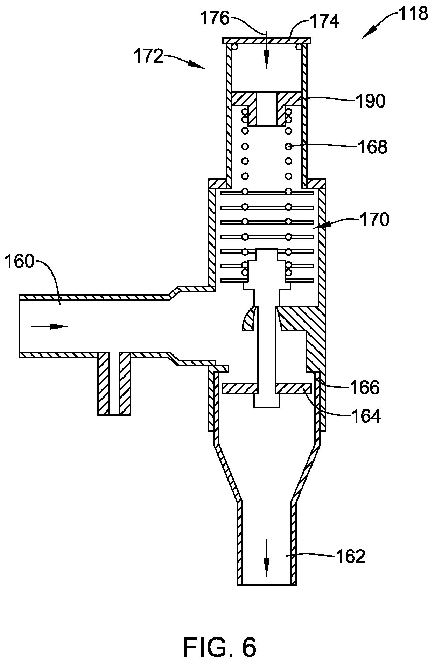

Suction may be started and stopped with the on/off valve 118. It is contemplated that the on/off valve 118 may help prevent leakages by stopping the flow of blood when flow is not desired. Further, the valve 118 may be adjusted to adjust the suction pressure. FIG. 6 is a cross-sectional view of an illustrative on/off valve 118. The valve 118 may include a fluid inlet 160 for receiving fluid from the catheter 102 and a fluid outlet 162. When the valve 118 is in an open configuration, as shown in FIG. 6, fluid may flow from the inlet 160 to the outlet 162. A valve seat disk 164 may be movable between an open configuration (FIG. 6) and a closed configuration (not explicitly shown). In the closed configuration, the valve seat disk 164 may rest against a valve seat 166 to prevent fluid from passing from the inlet 160 to the outlet 162.

The valve seat disk 164 may be connected to a spring 168 and a bellows 170. The spring 168 may be coupled to a pen click actuation mechanism 172 to open and close (unseat and seat) the valve seat disk 164. For example, each time a button 174 of the valve 118 is pushed down, as shown at arrow 176, the valve seat disk 164 is moved between the locked (closed) and unlocked (open) configuration. For example, if the valve seat disk is in the closed configuration, actuation of the button 174 will move the valve seat disk 164 down, release the suction pressure, and allow fluid to flow between the inlet 160 and the outlet 162. When the button 174 is pushed again, the valve seat disk 164 moves up and rests against the valve seat 166 and locking the suction pressure. In some cases, the position of the spring 168 can be adjusted with an adjustment mechanism 190. The adjustment mechanism 190 may be moved up or down to adjust the suction pressure, as desired.

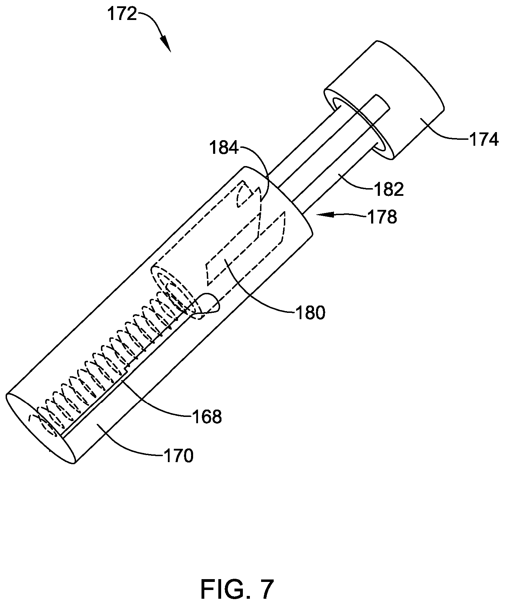

FIG. 7 is a perspective view of the illustrative actuation mechanism 172 of the valve 118. The spring 168 may be configured to bias the valve seat disk 164 to the closed configuration. However, the actuation mechanism 172 may be configured to override the spring 168 and push the valve seat disk 164 into the open configuration. The actuation mechanism 172 may include a cam or thrust device 178. The cam 178 may include a plurality of grooves 180, a plurality of slants 184, and a plurality of ledges 182. When the button 174 is depressed or clicked, the button 174 pushes the cam 178 against the force of the spring 168. The grooves 180, ledges 182, and slants 184 may cause the cam to rotate as it is pushed downwards 176. When the downward force is released, the spring 168 forces the cam 178 into one of two positions (closed or opened) based on the orientation of the mating grooves 180 and ledges 182. The grooves 180 and ledges 182 may be configured such that each time the button 174 is pressed to valve seat disk 164 moves to the opposing position. For example, if the valve seat disk 164 is in the opened configuration when the button 174 is depressed, the valve seat disk 164 will move to the closed configuration and vice versa.

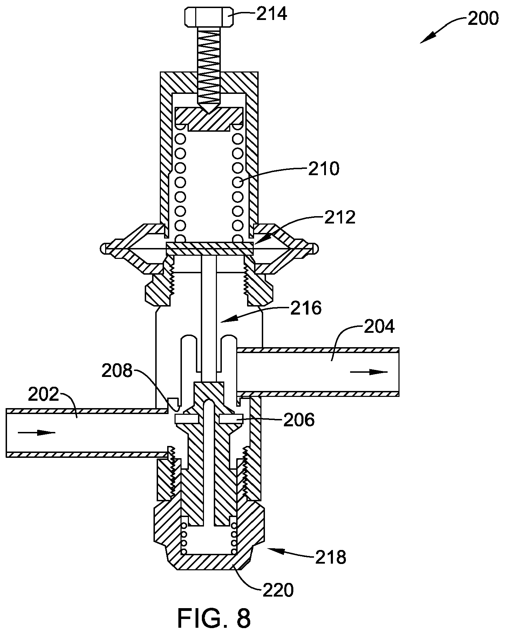

FIG. 8 is a cross-sectional view of another illustrative on/off valve 200. The valve 200 may include a fluid inlet 202 for receiving fluid from the catheter 102 and a fluid outlet 204. When the valve 200 is in an open configuration, as shown in FIG. 8, fluid may flow from the inlet 202 to the outlet 204. A valve seat disk 206 may be movable between an open configuration (FIG. 8) and a closed configuration (not explicitly shown). In the closed configuration, the valve seat disk 206 may rest against a valve seat 208 to prevent fluid from passing from the inlet 202 to the outlet 204.

The valve seat disk 206 may be coupled to an actuation mechanism 218 to open and close (unseat and seat) the valve seat disk 206. For example, a cap 220 of the valve 200 may be rotated in a first direction and a second direction. Depending on the direction of rotation, the valve seat disk 206 is moved between the locked (closed) and unlocked (open) configuration. The valve seat disk 206 may also be operably connected to a spring 210 and a diaphragm 212 via a push rod 216. An adjusting screw 214 may be positioned adjacent to the spring 210. The adjusting screw 214 may be configured to advance or retract to control a position of the spring 210 adjust how far the valve seat disk 206 can open and thus the suction pressure, as desired.

The materials that can be used for the various components of the suction device 10 and the thrombectomy system 100 (and/or other systems disclosed herein) and the various elements thereof disclosed herein may include those commonly associated with medical devices. For simplicity purposes, the following discussion makes reference to the suction device 10 and the thrombectomy system 100. However, this is not intended to limit the devices and methods described herein, as the discussion may be applied to other elements, members, components, or devices disclosed herein.

In some embodiments, suction device 10 and the thrombectomy system 100, and/or components thereof, may be made from a metal, metal alloy, polymer (some examples of which are disclosed below), a metal-polymer composite, ceramics, combinations thereof, and the like, or other suitable material.

Some examples of suitable polymers may include polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), Marlex high-density polyethylene, Marlex low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, biocompatible polymers, other suitable materials, or mixtures, combinations, copolymers thereof, polymer/metal composites, and the like.

Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R30035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY^{®}, PHYNOX^{®}, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear than the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Other suitable materials may include ULTANIUM^{™} (available from Neo-Metrics) and GUM METAL^{™} (available from Toyota). In some other embodiments, a superelastic alloy, for example a superelastic nitinol can be used to achieve desired properties.

In at least some embodiments, portions or all of the thrombectomy system 100, and/or components thereof, may also be doped with, made of, or otherwise include a radiopaque material. Radiopaque materials are understood to be materials capable of producing a relatively bright image on a fluoroscopy screen or another imaging technique during a medical procedure. This relatively bright image aids the user of the thrombectomy system 100 in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of the thrombectomy system 100 to achieve the same result.

In some embodiments, a degree of Magnetic Resonance Imaging (MRI) compatibility is imparted into the thrombectomy system 100. For example, the thrombectomy system 100, and/or components or portions thereof, may be made of a material that does not substantially distort the image and create substantial artifacts (e.g., gaps in the image). Certain ferromagnetic materials, for example, may not be suitable because they may create artifacts in an MRI image. The thrombectomy system 100, or portions thereof, may also be made from a material that the MRI machine can image. Some materials that exhibit these characteristics include, for example, tungsten, cobalt-chromium-molybdenum alloys (e.g., UNS: R30003 such as ELGILOY^{®}, PHYNOX^{®}, and the like), nickel-cobalt-chromium-molybdenum alloys (e.g., UNS: R30035 such as MP35-N^{®} and the like), nitinol, and the like, and others.

In some embodiments, an exterior surface of the suction device 10 and the thrombectomy system 100 (including, for example, an exterior surface of the delivery system) may be sandblasted, beadblasted, sodium bicarbonate-blasted, electropolished, etc. In these as well as in some other embodiments, a coating, for example a lubricious, a hydrophilic, a protective, or other type of coating may be applied over portions or all of the outer sheath, or in embodiments without an outer sheath over portions of the delivery system, or other portions of the thrombectomy system 100. Hydrophobic coatings such as fluoropolymers provide a dry lubricity which improves device handling and device exchanges. Lubricious coatings improve steerability and improve lesion crossing capability. Suitable lubricious polymers are well known in the art and may include silicone and the like, hydrophilic polymers such as high-density polyethylene (HDPE), polytetrafluoroethylene (PTFE), polyarylene oxides, polyvinylpyrolidones, polyvinylalcohols, hydroxy alkyl cellulosics, algins, saccharides, caprolactones, and the like, and mixtures and combinations thereof. Hydrophilic polymers may be blended among themselves or with formulated amounts of water insoluble compounds (including some polymers) to yield coatings with suitable lubricity, bonding, and solubility.

The coating and/or sheath may be formed, for example, by coating, extrusion, co-extrusion, interrupted layer co-extrusion (ILC), or fusing several segments end-to-end. The layer may have a uniform stiffness or a gradual reduction in stiffness from the proximal end to the distal end thereof. The gradual reduction in stiffness may be continuous as by ILC or may be stepped as by fusing together separate extruded tubular segments. The outer layer may be impregnated with a radiopaque filler material to facilitate radiographic visualization. Those skilled in the art will recognize that these materials can vary widely without deviating from the scope of the present invention.

It should be understood that this disclosure is, in many respects, only illustrative. Changes may be made in details, particularly in matters of shape, size, and arrangement of steps without exceeding the scope of the invention. This may include, to the extent that it is appropriate, the use of any of the features of one example embodiment being used in other embodiments. The invention's scope is, of course, defined in the language in which the appended claims are expressed.

## Claims

1. A suction device (10), comprising:
a barrel (12) defining a cavity (18) and extending from a first end (14) to a second end (16);
a first fluid inlet (20) adjacent the first end (14) of the barrel (12) and a second fluid inlet (24) adjacent the second end (16) of the barrel (12);
a first fluid outlet (22) adjacent the first end (14) of the barrel (12) and a second fluid outlet (26) adjacent the second end (16) of the barrel (12);
a plunger (28) slideably disposed within the cavity (18) of the barrel (12); and
a handle assembly (30) operably coupled to the plunger (28);
wherein actuation of the handle assembly (30) is configured to move the plunger (28) within the cavity (18) between the first end (14) and the second end (16) of the barrel (12); **characterised in that** the handle assembly (30) comprises a moveable first handle member (32) pivotably coupled to a second handle member (34), and a connecting member (36) extending between the first handle member (32) and the second handle member (34); wherein the second handle member (34) is fixedly coupled to the connecting member (36); and wherein the connecting member (36) is fixedly secured to the barrel (12).

2. The suction device of claim 1, wherein a portion of the first handle member (32) is moveably disposed within a slot (46) of the connecting member (36).

3. The suction device of claim 1 or claim 2, further comprising a biasing mechanism (64) disposed within the connecting member (36).

4. The suction device of claim 3, wherein the biasing mechanism (64) is configured to bias the first handle member (32) towards the first end (14) of the barrel (12).

5. The suction device of claim 4, wherein actuation of the first handle member (32) is configured to move the plunger (28) in a first direction and the biasing mechanism (64) is configured to move the plunger (28) in a second direction opposite the first direction.

6. The suction device of any one of claims 1-4, wherein the plunger (28) includes a magnetic material.

7. The suction device of claim 6, wherein the handle assembly (30) is magnetically coupled to the plunger (28).

8. The suction device of claim 7, wherein the handle assembly (30) comprises an annular portion disposed about an outer perimeter of the barrel (12).

9. The suction device of claim 8, wherein the annular portion includes a magnetic material.

10. The suction device of any one of claims 1-8, wherein movement of the plunger (28) within the cavity (18) is configured to simultaneously draw fluid in through at least one of the first and second inlets (20, 24) and expel fluid through at least one of the first and second outlets (22, 26).

11. The suction device of any one of claims 1-9, wherein the plunger (28) forms a fluid tight seal with an interior surface of the barrel (12).

## Patentansprüche

1. Saugvorrichtung (10), aufweisend:
ein Rohr (12), das einen Hohlraum (18) definiert und sich von einem ersten Ende (14) zu einem zweiten Ende (16) erstreckt;
einen ersten Fluideinlass (20) angrenzend an das erste Ende (14) des Rohrs (12) und einen zweiten Fluideinlass (24) angrenzend an das zweite Ende (16) des Rohrs (12);
einen ersten Fluidauslass (22) angrenzend an das erste Ende (14) des Rohrs (12) und einen zweiten Fluidauslass (26) angrenzend an das zweite Ende (16) des Rohrs (12);
einen Kolben (28), der gleitbar in dem Hohlraum (18) des Rohrs (12) angeordnet ist; und
eine Griffanordnung (30), die operativ mit dem Kolben (28) gekoppelt ist;
wobei eine Betätigung der Griffanordnung (30) konfiguriert ist, den Kolben (28) in dem Hohlraum (18) zwischen dem ersten Ende (14) und dem zweiten Ende (16) des Rohrs (12) zu bewegen;
**dadurch gekennzeichnet, dass** die Griffanordnung (30) ein bewegbares erstes Griffelement (32), das drehbar mit einem zweiten Griffelement (34) gekoppelt ist, und ein sich zwischen dem ersten Griffelement (32) und dem zweiten Griffelement (34) erstreckendes Verbindungselement (36) aufweist, wobei das zweite Griffelement (34) starr mit dem Verbindungselement (36) gekoppelt ist; und wobei das Verbindungselement (36) starr an dem Rohr (12) befestigt ist.

2. Saugvorrichtung nach Anspruch 1, wobei ein Teil des ersten Griffelements (32) bewegbar in einem Schlitz (46) des Verbindungselements (36) angeordnet ist.

3. Saugvorrichtung nach Anspruch 1 oder Anspruch 2, ferner aufweisend einen Vorspannungsmechanismus (64), der in dem Verbindungselement (36) angeordnet ist.

4. Saugvorrichtung nach Anspruch 3, wobei der Vorspannungsmechanismus (64) konfiguriert ist, das erste Griffelement (32) hin zu dem ersten Ende (14) des Rohrs (12) vorzuspannen.

5. Saugvorrichtung nach Anspruch 4, wobei eine Betätigung des ersten Griffelements (32) konfiguriert ist, den Kolben (28) in eine erste Richtung zu bewegen, und der Vorspannungsmechanismus (64) konfiguriert ist, den Kolben (28) in eine zur ersten Richtung entgegengesetzte zweite Richtung zu bewegen.

6. Saugvorrichtung nach einem der Ansprüche 1 bis 4, wobei der Kolben (28) ein magnetisches Material aufweist.

7. Saugvorrichtung nach Anspruch 6, wobei die Griffanordnung (30) magnetisch mit dem Kolben (28) gekoppelt ist.

8. Saugvorrichtung nach Anspruch 7, wobei die Griffanordnung (30) einen ringförmigen Teil aufweist, der um einen Außenumfang des Rohrs (12) angeordnet ist.

9. Saugvorrichtung nach Anspruch 8, wobei der ringförmige Teil ein magnetisches Material aufweist.

10. Saugvorrichtung nach einem der Ansprüche 1 bis 8, wobei eine Bewegung des Kolbens (28) in dem Hohlraum (18) konfiguriert ist, Fluid durch mindestens einen des ersten und zweiten Einlasses (20, 24) einzusaugen und geleichzeitig Fluid durch mindestens einen des ersten und zweiten Auslasses (22, 26) auszustoßen.

11. Saugvorrichtung nach einem der Ansprüche 1 bis 9, wobei der Kolben (28) eine fluiddichte Abdichtung mit einer Innenfläche des Rohrs (12) bildet.

## Revendications

1. Dispositif d'aspiration (10) comprenant :
un tuyau (12) définissant une cavité (18) et s'étendant à partir d'une première extrémité (14) jusqu'à une deuxième extrémité (16) ;
une première entrée de fluide (20) adjacente à la première extrémité (14) du tuyau (12) et une deuxième entrée de fluide (24) adjacente à la deuxième extrémité (16) du tuyau (12) ;
une première sortie de fluide (22) adjacente à la première extrémité (14) du tuyau (12) et une deuxième sortie de fluide (26) adjacente à la deuxième extrémité (16) du tuyau (12) ;
un piston plongeur (28) disposé, de manière coulissante, à l'intérieur de la cavité (18) du tuyau (12) ; et
un ensemble de poignée (30) couplé, de manière opérationnelle, au piston plongeur (28) ;
dans lequel l'actionnement de l'ensemble de poignée (30) est configuré pour déplacer le piston plongeur (28) à l'intérieur de la cavité (18) entre la première extrémité (14) et la deuxième extrémité (16) du tuyau (12) ; **caractérisé en ce que** :
l'ensemble de poignée (30) comprend un premier élément de poignée (32) mobile couplé, de manière pivotante, à un deuxième élément de poignée (34), et un élément de raccordement (36) s'étendant entre le premier élément de poignée (32) et le deuxième élément de poignée (34) ; dans lequel le deuxième élément de poignée (34) est couplé, de manière fixe, à l'élément de raccordement (36) ; et dans lequel l'élément de raccordement (36) est fixé, de manière fixe, au tuyau (12).

2. Dispositif d'aspiration selon la revendication 1, dans lequel une partie du premier élément de poignée (32) est disposée, de manière mobile, à l'intérieur d'une fente (46) de l'élément de raccordement (36).

3. Dispositif d'aspiration selon la revendication 1 ou la revendication 2, comprenant en outre un mécanisme de sollicitation (64) disposé à l'intérieur de l'élément de raccordement (36).

4. Dispositif d'aspiration selon la revendication 3, dans lequel le mécanisme de sollicitation (64) est configuré pour solliciter le premier élément de poignée (32) vers la première extrémité (14) du tuyau (12).

5. Dispositif d'aspiration selon la revendication 4, dans lequel l'actionnement du premier élément de poignée (32) est configuré pour déplacer le piston plongeur (28) dans une première direction et le mécanisme de sollicitation (64) est configuré pour déplacer le piston plongeur (28) dans une deuxième direction opposée à la première direction.

6. Dispositif d'aspiration selon l'une quelconque des revendications 1 à 4, dans lequel le piston plongeur (28) comprend un matériau magnétique.

7. Dispositif d'aspiration selon la revendication 6, dans lequel l'ensemble de poignée (30) est couplé, par voie magnétique, au piston plongeur (28).

8. Dispositif d'aspiration selon la revendication 7, dans lequel l'ensemble de poignée (30) comprend une partie annulaire disposée autour d'un périmètre externe du tuyau (12).

9. Dispositif d'aspiration selon la revendication 8, dans lequel la partie annulaire comprend un matériau magnétique.

10. Dispositif d'aspiration selon l'une quelconque des revendications 1 à 8, dans lequel le déplacement du piston plongeur (28) à l'intérieur de la cavité (18) est configuré pour aspirer simultanément le fluide à travers au moins l'une des première et deuxième entrées (20, 24) et pour expulser le fluide par au moins l'une des première et deuxième sorties (22, 26).

11. Dispositif d'aspiration selon l'une quelconque des revendications 1 à 9, dans lequel le piston plongeur (28) forme un joint d'étanchéité étanche au fluide avec une surface intérieure du tuyau (12).
